# EUROPEAN PATENT SPECIFICATION

(11) **EP 4 284 458 B1**
(45) Date of publication and mention of the grant of the patent: **23.10.2024**
(21) Application number: 22701316.6
(22) Date of filing: 11.01.2022
(51) Int. Cl.: A61L 31/10

(54) **RAPIDLY RESORBABLE INTRAVASCULAR IMPLANT**
SCHNELL RESORBIERBARES INTRAVASKULÄRES IMPLANTAT
IMPLANT INTRAVASCULAIRE RAPIDEMENT RÉSORBABLE

(30) Priority: 29.01.2021 DE 202021100450 U
(43) Date of publication of application: 06.12.2023
(73) Proprietor: BIOTRONIK AG, 8180 Bülach (CH)
(72) Inventor: MÜLLER, Heinz, 18055 Rostock (DE); ZUMSTEIN, Marion Philine, 8810 Horgen (CH)
(74) Representative: Biotronik Corporate Services SE
(86) International application number: PCT/EP2022/050465
(87) International publication number: WO 2022/161762

(56) References cited:
- US-A1- 2010 076 544
- US-A1- 2010 076 556
- US-A1- 2014 199 365

## Description

The invention relates to an implant in the form of a vascular support. Such a vascular support is also referred to as a scaffold and is generally designed as a circumferential structure made of struts that are connected to one another and form the cells of the scaffold.

Rapidly biodegradable vascular supports for sealing vulnerable plaques or biodegradable vascular supports as temporary flow diverters for treating aneurysms are not available in the market.

If permanent or slowly degradable vascular supports are used for this purpose, even though an implant is only required for a very short period of time, the implant (for example, due to potential thromboses) or necessary long-term medication (for example, additional hemorrhage due to the administration of thrombocyte aggregation inhibitors) poses a potential longer-term or permanent risk in the least favorable case, for example in the case of intracranial aneurysms with permanent implants.

The biodegradable vascular supports/scaffolds available in the market were primarily developed to preserve as great a radial force as possible for the longest possible time, which is not even required for the above-described applications.

The disadvantages of these implants, which are predominantly made of plastic (PLLA), are, in principle, high wall thicknesses, in many instances poor adaptation to the shape of the vessel (malapposition), very long degradation times and, resulting from all these factors, a relative high risk of thrombosis.

Mg-based vascular supports additionally result in a permanent degradation product; however, the tendency to cause thrombosis appears to be drastically reduced compared to permanent metals and plastic materials, regardless of the wall thickness.

US 2014/199365 A1 is directed to a stent made of a magnesium alloy degradable under physiological conditions and an outer polymeric coating that allows to control the degradation of the stent. The biologically degradable magnesium alloy contains 5-25.5 wt% dysprosium, 0.01-5 wt% neodynium and/or europium, 0.1-3 wt% zinc, 0.1-2 wt% zirconium , balance to 100 wt% magnesium wherein the stent has a polymeric coating. The polymer coating comprises or consists of one or several substances selected from the group consisting of one of several substances selected from a list including polylactides, polyglycolides, polyanhydrides, polyhydroxybutyrate, polycaprolactone, polydioxanone. After the entire dissolution of the inner basic scaffold, the polymeric coating can also be degraded biologically, so that after a few months the stent has entirely dissolved.

Proceeding from this, the object underlying the invention is to provide an implant or a vascular support that is at least partially improved with respect to the aforementioned disadvantages, and additionally shows substantially residue-free degradation. In particular, a rapidly biodegradable vascular support is to be provided, which has mechanical integrity that is limited in terms of time and which can be degraded after the mechanical integrity has been lost, and preferably is used to seal vulnerable plaques or as a temporary flow diverter for treating aneurysms.

This object is achieved by an implant, in particular a vascular support, having the features of claim 1. Advantageous embodiments of the invention will be described hereafter.

According to claim 1, an implant is disclosed, in particular a vascular support, in particular for implantation into a vulnerable plaque or for disrupting the flow into an aneurysm, wherein the implant can be fully biodegraded within a time period of less than 360 days, in particular of less than 180 days, preferably of less than 90 days, wherein the implant in particular comprises a magnesium alloy or is made thereof, including Zn (zinc) and Ca (calcium).

The invention thus in particular provides an implant or a scaffold/vascular support that can be implanted into an existing vulnerable plaque, so that uncontrolled rupturing of the plaques, and a potentially subsequent thrombotic event, can be prevented, and the implant can, thereafter, be completely biodegraded within a time period of less than 360 days, preferably of less than 180 days, and ideally in less than 90 days.

Furthermore, one embodiment of the invention relates to an implant or a vascular support/scaffold that, when an aneurysm exists, disrupts the flow into the arising cavity which maintains this aneurysm from a fluid point of view, so that a flow-free zone can arise in the space of the former aneurysm and the cavity can be incrementally closed by coagulation and cell colonization. In the process, depending on the requirements of the therapy, the implant is to be completely dissolved after a time period of less than 360 days, less than 180 days, or even less than 90 days, so that the risk of thrombosis caused by a foreign object or a degradation product is avoided, especially in intracranial vessels. The invention relates in particular to an implant for treating local vascular diseases that require only short-term mechanical stabilization (for example vulnerable plaques or aneurysms). According to one embodiment of the invention, it is provided that the implant is made of or comprises a biodegradable magnesium alloy, in which the time until full biodegradation can be varied by way of the content of the involved alloying elements. The implant (for example vascular support) can be coated with a polymer, and this polymer can contain a pharmaceutical drug.

As a result of rapid degradation of an implant in the two applications mentioned above, the time during which a patient has to be treated with thrombocyte aggregation inhibitors (dual antiplatelet therapy, DAPT) is also decreased. A shorter therapy duration reduces the risk of undesirable hemorrhage. In addition, minimal and only brief mechanical loading of the vessel decreases a provocation of the excess proliferation of neointima and potential stenosing of the affected vascular section associated therewith.

According to the invention, a biodegradable vascular support is provided, which can be implanted into the vascular wall at the appropriate location by way of a balloon. Such a vascular support is made of a biodegradable metal. To achieve complete dissolution after less than 360 days, a magnesium alloy (Mg alloy for short) is used for this purpose. The Mg alloys are those alloys that contain Zn and optionally Ca.

Surprisingly, it was additionally shown in animal experiments that this group of alloys can be degraded to a very large degree after a comparatively shorter implantation duration, and can be fully degraded in the case of the more rapidly degrading alloys that have higher Zn contents, without leaving behind a degradation product or a relevant amount of residues.

This finding was not to be expected insofar as other previously examined alloys, specifically magnesium alloys, that contain rare earths or aluminum as the alloying elements have a longer degradation time, and leave a degradation product behind.

The degradation kinetics can be set by way of the Zn content or by way of the Zn/Ca ratio, in general the following applying:
- the higher the Zn content, the more rapidly the degradation progresses;
- the lower the Zn content, the more slowly the degradation progresses;
- the higher the Zn/Ca ratio, the more rapidly the degradation progresses;
- the lower the Zn/Ca ratio, the more slowly the degradation progresses.

According to the invention, it is provided that the Mg alloy is a Mg alloy having a Zn content between 1.5 wt.% and 20.0 wt.% Zn and comprising 0 wt.% to 1 wt.% Ca.

According to a further embodiment, preferably Mg alloys comprising between 1.5 wt.% and 6.0 wt.% Zn are to be selected, in particular for full thermomechanical processability, since all the Zn can be brought into solution at up to 6.0 wt.% Zn.

According to a further embodiment, Mg alloys having a Zn content of ≥ 1.5 wt.% and a Ca content of < 0.5 wt.% are preferred, in particular for rapid and substantially reliable full dissolution.

According to a further embodiment of the invention, it is provided that Mg alloys comprising more than 6.0 wt.% Zn are to be selected, in particular for the high strength possible, since here a permanently precipitated intermetallic phase of Mg and Zn is present, and very strong particle hardening is also achieved at room temperature due to the high percent by volume.

The above-described Mg alloys are also referred to as high Zn alloys hereafter. These alloys are in particular suitable for achieving as complete a degradation as possible in less than 360 days, possible also in considerably less time, as was able to be demonstrated in animal experiments.

If it is desired that an implant according to the invention is to be stable initially for a certain period of time, but is to degrade as rapidly as possible thereafter, it is provided, according to a further embodiment, to additionally coat the respective high Zn alloy or the implant made thereof with a biodegradable polymer. In this case, for example, an implant in the form of a vascular support is initially protected by the polymer against corrosion. Following degradation of the polymer, which has progressed far enough for the physiological medium to penetrate the layer, the vascular support or the implant degrades, which takes place particularly rapidly in the case of the high Zn alloys. Suitable biodegradable polymers are listed below.

If it is desired to optimize the mechanical properties of one of the aforementioned metal alloys (for example by way of grain refinement) or to further expedite the degradation kinetics thereof, for example for reasons related to the mechanical properties, when the composition being is fixedly defined, this can be achieved, according to a further embodiment of the invention, by adding one or more micro-alloying elements.

According to one embodiment, such micro-alloying elements are typically used in contents of less than 1 wt.%, preferably in contents of 0.01 wt.% to 0.1 wt.%.

This can preferably involve the addition of one or more of the elements Ag, Fe, Mn, Si, for example, wherein in particular Mn and Si, preferably in combination, can be used to generate specific, strength-enhancing and corrosion-expediting intermetallic phases.

This definition of limits for the contents of possible micro-alloying elements applies to both alloy types.

To obtain particularly advantageous mechanical properties, one embodiment of the implant according to the invention is provided, which has a fine-grained microstructure, having a grain size of no more than 7.5 µm, preferably < 5 µm, and in particular preferably < 2.5 µm. During the dilatation of plaques, particles (for example components of the soft, fat-like plaques or also small, fully or partially calcified particles) can be released. To additionally offer particularly high protection against such a possible release of particles, the vascular support is additionally provided with a net. This net can be attached to the inside or outside.

According to one embodiment, the net is made of one of the aforementioned biodegradable metal alloys, wherein a preferred diameter of the wires that are used ranges between 10 µm and 100 µm, and/or wherein the preferred size of the mesh (distance between the wires) ranges between 10 µm and 1 mm.

According to one embodiment, the net is made of one of the aforementioned biodegradable metal alloys, wherein a preferred diameter of the wires that are used ranges between 10 µm and 100 µm, and/or wherein the preferred size of the mesh (distance between the wires) ranges between 10 µm and 1 mm, wherein the net is additionally coated with a biodegradable polymer.

At least one of the following polymers can be used as the biodegradable polymer, which can be used as a coating on the resorbable base body of the implant, in particular of the vascular support, within the above-described meaning: polylactide, polyglycolide, polyanhydride, polyhydroxybutyrate, polycaprolactone, polydioxanone, a poly(trimethylene carbonate)-based polymer, polyphosphazene, polyhydroxyalkonates, polyanhydride, polyacetal, polycarbonate, poly(ether ester); a copolymer of the aforementioned polymers, a mixture of the aforementioned polymers, or a blend of the aforementioned polymers.

The following are considered to be particularly suitable: poly(L-lactide), poly)D,L-lactide), poly(D,L-lactide-co-glycolide), polyhydroxybutyrate, polycaprolactone, copolymers of the aforementioned polymers.

According to one embodiment of the invention, the layer thickness of the polymer layers can be 50 nm to 25 µm, layer thicknesses of 200 nm to 10 µm being preferred, and those of 1 µm to 5 µm being particularly preferred.

To be able to achieve as effective covering of a vulnerable plaque or of an aneurysm as possible by way of the base body of the vascular support, additional designs can optionally be preferred for the vascular supports which have an increased so-called "metal to artery ratio" compared to conventional vascular supports. The metal to artery ratio describes the percentage of the vessel wall that is covered over the length across which the implant extends in the vessel. Suitable metal to artery ratios for implants or vascular supports according to the invention are in a range of above 15%, preferably above 25%, and in cases in which the greatest possible coverage is desired, up to 50%. (The information always relates to the coverage in percent after implantation).

Further examples of the invention using high Zn alloys
- Vascular support made of a MgZn5Ca0.25 alloy for dilating a vulnerable plaque in a coronary vessel, of which ≥ 95% of the volume of the body is degraded residue-free within approximately 90 days; the vessel support has a metal to artery ratio of 25% and a 3 µm thick layer made of polylactide.
- Vascular support made of a MgZn2.5Ca0.5 alloy, which is used as a flow diverter for an intracranial aneurysm, can maintain the geometric integrity thereof for ≥ 2 weeks, and of which ≥ 80% of the volume of the body is degraded residue-free within approximately 180 days, wherein the vascular support is furthermore coated with a layer made of a polylactide/polycaprolactone 70/30 blend.
- Vascular support made of a MgZn4 alloy, which includes a woven net made of wires of a MgZn2 alloy on the inside, for dilating a vulnerable plaque in a coronary vessel, wherein the net, which is made of wires having a thickness of 20 µm to 50 µm and a mean distance between the wires of approximately 250 µm, prevents components of the vulnerable plaque from reaching the coronary vessel in the event the plaque ruptures.
- Vascular support having high stability, made of a MgZn7.5 alloy for sealing a vulnerable plaque in a coronary vessel, which can be used uncoated or containing a layer made of polyhydroxybutyrate; the vessel support has a metal to artery ratio of 35%.

The advantage of the solution according to the invention is that an implant can be provided for the above-described applications which can dissolve rapidly and completely, without residue or with an extremely small amount of degradation products that remain, within a period of time of less than 360 days, or also within a considerably shorter period of time, and thereby avoids undesirable long-term complications or the need for drug therapies, for example for anti-coagulation.

## Claims

1. An implant, in the form of a vascular support, in particular for implantation into a vulnerable plaque or for disrupting flow to an aneurysm, wherein the implant can be fully biodegraded within a time period of less than 360 days, in particular less than 180 days, preferably less than 90 days, and wherein the implant in particular comprises a magnesium alloy or is made thereof, wherein the Mg alloy is a Mg alloy having a Zn content between 1.5 wt.% and 20.0 wt.% Zn and comprising 0 wt.% to 1 wt.% Ca, **characterized in that** the implant is provided with a net being made of one of the aforementioned biodegradable metal alloys and having wires, wherein the diameter of the wires that are used ranges between 10 µm and 100 µm.

2. The implant of claim 1, wherein one or more micro-alloying elements are added to the alloy.

3. The implant of claim 2, wherein the micro-alloying elements are used in contents of less than 1 wt.%, and preferably in contents of 0.01 wt.% to 0.1 wt.%.

4. The implant of claim 2 or 3, wherein the micro-alloying elements are one or more of the elements Ag, Fe, Mn and Si.

5. The implant of one of the preceding claims, wherein the implant has a fine-grained microstructure, having a grain size of no more than 7.5 µm, preferably < 5 µm, and in particular preferably < 2.5 µm.

6. The implant of one of the preceding claims, wherein the net is attached to the inside or outside of the implant.

7. The implant of claim 1 or 6, wherein the distance between the wires ranges between 10 µm and 1 mm.

8. The implant of claim 1 or 6 or 7, wherein the net is additionally coated with a biodegradable polymer.

9. The implant of claim 8, wherein the polymer is selected from the group consisting of polylactide, polyglycolide, polyanhydride, polyhydroxybutyrate, polycaprolactone, polydioxanone, a poly(trimethylene carbonate)-based polymer, polyphosphazene, polyhydroxyalkonates, polyanhydride, polyacetal, polycarbonate, poly(ether ester); a copolymer of the aforementioned polymers, a mixture of the aforementioned polymers, or a blend of the aforementioned polymers.

10. The implant of claim 8 or 9, wherein the layer thickness of the polymer layers is 50 nm to 25 µm.

## Patentansprüche

1. Implantat in Form einer Gefäßstütze, insbesondere zur Implantation in eine vulnerable Plaque oder zur Unterbrechung eines Stroms zu einem Aneurysma, wobei das Implantat innerhalb eines Zeitraums von weniger als 360 Tagen, insbesondere weniger als 180 Tagen, vorzugsweise weniger als 90 Tagen, vollständig biologisch abgebaut werden kann, und wobei das Implantat insbesondere eine Magnesiumlegierung umfasst oder aus einer solchen gefertigt ist, wobei die Mg-Legierung eine Mg-Legierung mit einem Zn-Gehalt zwischen 1,5 Gew.-% und 20 Gew.-% Zn ist und 0 Gew.-% bis 1 Gew.-% Ca umfasst, **dadurch gekennzeichnet, dass** das Implantat mit einem Netz versehen ist, das aus einer der genannten biologisch abbaubaren Metalllegierungen gefertigt ist und Drähte umfasst, wobei der Durchmesser der verwendeten Drähte in einem Bereich zwischen 10 µm und 100 µm liegt.

2. Implantat nach Anspruch 1, wobei der Legierung ein oder mehrere Mikrolegierungselemente zugesetzt sind.

3. Implantat nach Anspruch 2, wobei Gehalte an verwendeten Mikrolegierungselementen weniger als 1 Gew.-% und vorzugsweise 0,01 Gew.-% bis 0,1 Gew.-% betragen.

4. Implantat nach Anspruch 2 oder 3, wobei es sich bei den Mikrolegierungselementen um eines oder mehrere von den Elementen Ag, Fe, Mn und Si handelt.

5. Implantat nach einem der vorangehenden Ansprüche, wobei das Implantat eine feinkörnige Mikrostruktur hat, mit einer Korngröße von nicht mehr als 7,5 µm, vorzugsweise < 5 µm, und besonders bevorzugt von < 2,5 µm.

6. Implantat nach einem der vorangehenden Ansprüche, wobei das Netz an der Innenseite oder der Außenseite des Implantats angebracht ist.

7. Implantat nach Anspruch 1 oder 6, wobei der Abstand zwischen den Drähten in einem Bereich zwischen 10 µm und 1 mm liegt.

8. Implantat nach Anspruch 1 oder 6 oder 7, wobei das Netz zusätzlich mit einem biologisch abbaubaren Polymer beschichtet ist.

9. Implantat nach Anspruch 8, wobei das Polymer ausgewählt ist aus der Gruppe bestehend aus Polylactid, Polyglykolid, Polyanhydrid, Polyhydroxybutyrat, Polycaprolacton, Polydioxanon, einem Polymer auf Poly(trimethylencarbonat)-Basis, Polyphosphazen, Polyhydroxyalkonaten, Polyanhydrid, Polyacetal, Polycarbonat, Poly(etherester); einem Copolymer der genannten Polymere, einer Mischung der genannten Polymere oder einem Blend der genannten Polymere.

10. Implantat nach Anspruch 8 oder 9, wobei die Schichtdicke der Polymerschichten 50 nm bis 25 µm beträgt.

## Revendications

1. Prothèse, sous la forme d'un support vasculaire, en particulier pour implantation dans une plaque vulnérable ou pour perturber le débit vers un anévrisme, dans laquelle la prothèse peut être entièrement biodégradée dans une période de moins de 360 jours, en particulier de moins de 180 j ours, préférentiellement de moins de 90 jours, et dans laquelle la prothèse en particulier comprend un alliage de magnésium ou est fabriquée en celui-ci, dans laquelle l'alliage de Mg est un alliage de Mg ayant une teneur en Zn entre 1,5 % en masse et 20,0 % en masse de Zn et comprenant 0 % en masse à 1 % en masse de Ca, **caractérisée en ce que** la prothèse est pourvue d'un filet fabriqué en l'un des alliages métalliques biodégradables susmentionnés et ayant des fils, dans laquelle le diamètre des fils qui sont utilisés est compris entre 10 µm et 100 µm.

2. Prothèse selon la revendication 1, dans laquelle un ou plusieurs éléments de micro-alliage sont ajoutés à l'alliage.

3. Prothèse selon la revendication 2, dans laquelle les éléments de micro-alliage sont utilisés à des teneurs de moins de 1 % en masse, et préférentiellement à des teneurs de 0,01 % en masse à 0,1 % en masse.

4. Prothèse selon la revendication 2 ou 3, dans laquelle les éléments de micro-alliage sont un ou plusieurs parmi les éléments Ag, Fe, Mn et Si.

5. Prothèse selon l'une des revendications précédentes, dans laquelle la prothèse a une microstructure à grains fins, ayant une taille de grain ne dépassant pas 7,5 µm, préférentiellement < 5 µm, et en particulier préférentiellement < 2,5 µm.

6. Prothèse selon l'une des revendications précédentes, dans laquelle le filet est fixé à l'intérieur ou à l'extérieur de la prothèse.

7. Prothèse selon la revendication 1 ou 6, dans laquelle la distance entre les fils est comprise entre 10 µm et 1 mm.

8. Prothèse selon la revendication 1 ou 6 ou 7, dans laquelle le filet est de plus revêtu d'un polymère biodégradable.

9. Prothèse selon la revendication 8, dans laquelle le polymère est choisi dans le groupe constitué par le polylactide, le polyglycolide, le polyanhydride, le polyhydroxybutyrate, la polycaprolactone, la polydioxanone, un polymère à base de poly(triméthylène carbonate), le polyphosphazène, les polyhydroxyalconates, le polyanhydride, le polyacétal, le polycarbonate, le poly(éther ester) ; un copolymère des polymères susmentionnés, un mélange des polymères susmentionnés, ou une formulation des polymères susmentionnés.

10. Prothèse selon la revendication 8 ou 9, dans laquelle l'épaisseur de couche des couches de polymère est de 50 nm à 25 µm.
